# EUROPÄISCHE PATENTSCHRIFT

(11) **EP 0 687 258 B1**
(45) Veröffentlichungstag und Bekanntmachung des Hinweises auf die Patenterteilung: **22.07.1998**
(21) Anmeldenummer: 94908997.3
(22) Anmeldetag: 18.02.1994
(51) Int. Cl.: C07D 295/22, C07C 255/66, A61K 31/495, A61K 31/54, A61K 31/40, A61K 31/275

(54) **SUBSTITUIERTE IMINOACETONITRILE MIT MYOKARDPROTEKTIVER WIRKUNG**
SUBSTITUTED IMINOACETONITRILES WITH MYOCARDIAL PROTECTIVE EFFECT
IMINOACETONITRILES SUBSTITUES AYANT UN EFFET PROTECTEUR DU MYOCARDE

(30) Priorität: 04.03.1993 DE 4306738
(43) Veröffentlichungstag der Anmeldung: 20.12.1995
(73) Patentinhaber: HOECHST AKTIENGESELLSCHAFT, 65926 Frankfurt am Main (DE)
(72) Erfinder: PIPER, Hans, Michael, D-40723 Hilden (DE); SCHLÜTER, Klaus-Dieter, D-40591 Düsseldorf (DE); WEBER, Martin, D-59872 Meschede (DE); SCHÖNAFINGER, Karl, D-63755 Alzenau (DE); BRENDEL, Joachim, D-61118 Bad Vilbel (DE); SCHRAVEN, Eckhard, D-60386 Frankfurt am Main (DE)
(86) Internationale Anmeldenummer: EP9400460
(87) Internationale Veröffentlichungsnummer: WO9420482

(56) Entgegenhaltungen:
- DE-A- 3 113 222
- JOURNAL OF CARDIOVASCULAR PHARMACOLOGY Bd. 14, Nr. 11 , 1989 , NEW YORK Seiten S6 - S12 HELMUT BOHN AND KARL SCHÖNAFINGER in der Anmeldung erwähnt

## Beschreibung

Die vorliegende Erfindung betrifft substituierte Iminoacetonitrile, Verfahren zu ihrer Herstellung und ihre Verwendung in Arzneimitteln mit myokardprotektiver Wirkung.

Nitrosohydrazine der Formel 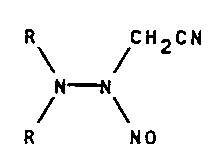 sind als wirksame Metabolite von Sydnoniminen beschrieben. Sie sind in der Lage, unter der Einwirkung von Sauerstoff oder Oxidationsmitteln die vasorelaxierende und plättchenfunktionshemmende Substanz Stickstoffmonoxid freizusetzen (siehe H. Bohn und K. Schönafinger

J. Cardiovasc. Pharmacol. 14 Suppl. 11: S 6 - S 12 (1989)). Als Produkt dieser NO-Freisetzung ist im Falle des Handelsprodukts Molsidomin auch bereits N-Morpholino-iminoacetonitril beschrieben, wobei aber pharmakologische Wirkungen dieser Verbindung bisher nicht bekannt waren. Es wurde nun überraschenderweise gefunden, daß Iminoacetonitrile dieser Art myokardprotektive Wirkungen besitzen, die ähnlich dem bereits bekannten Butandionmonoxim (BDM) durch eine Beeinflussung des Energiestoffwechsels der Myokardzelle zustande kommt. Die vorliegende Erfindung betrifft somit Verbindungen der allgemeinen Formel I 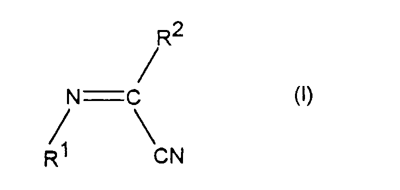 worin
- R¹: eine Aminogruppe der Formel 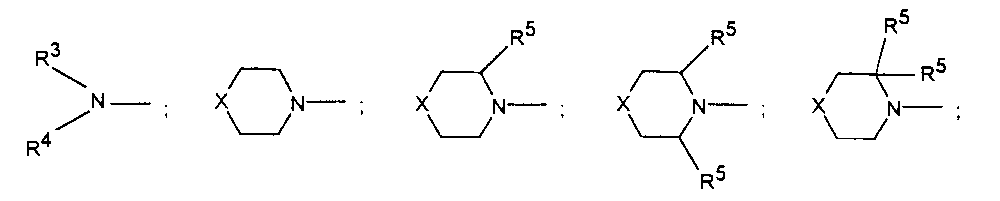 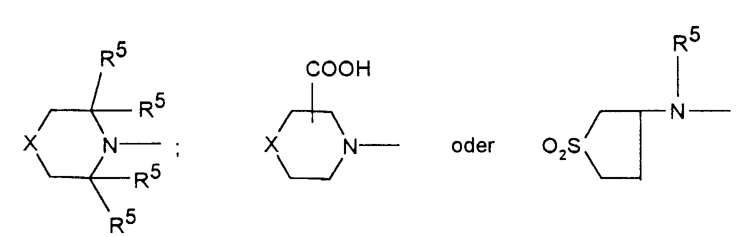 bedeutet;
- R²: Wasserstoff, Halogen, (C₁-C₆)-Alkyl, (C₅-C₇)-Cycloalkyl, (C₆-C₁₀)-Aryl-(C₁-C₆)-alkyl, (C₆-C₁₀)-Aryl-O-(C₁-C₆)-alkyl, (C₆-C₁₀)-Aryl-S-(C₁-C₆)-alkyl, (C₁-C₆)-Alkoxy-(C₁-C₆)-alkyl, (C₁-C₆)-Alkylthio-(C₁-C₆)-alkyl oder (C₁-C₆)-Alkenylthio-(C₁-C₆)-alkyl bedeutet;
- R³: (C₁-C₆)-Alkyl, (C₁-C₆)-Alkenyl, (C₅-C₇)-Cycloalkyl, (C₇-C₁₄)-Bicycloalkyl, (C₇-C₁₆)-Tricycloalkyl, (C₁-C₆)-Alkyl-X-(C₁-C₆)-alkyl, (C₆-C₁₀)-Aryl-(C₁-C₆)-alkyl, das auch durch (C₁-C₆)-Alkyl oder Halogen substituiert sein kann, Hydroxy-(C₁-C₆)-alkyl oder einen Rest 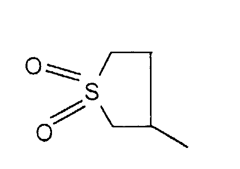
bedeutet;
- R⁴: Wasserstoff bedeutet oder eine der Bedeutungen von R³ hat;
- R⁵: (C₁-C₆)-Alkyl bedeutet;
- X: NR⁴, NSO₂R⁶, NCO₂-(C₁-C₆)-Alkyl, S(O)ₙ, O, (CH₂)ₘ oder eine einfache Bindung bedeutet;
- n: 0, 1 oder 2 ist;
- m: 1, 2 oder 3 ist; und
- R⁶: (C₁-C₆)-Alkyl, (C₆-C₁₀)-Aryl, (C₁-C₆)-Alkyl-(C₆-C₁₀)-aryl, Halogen-(C₆-C₁₀)-aryl oder Di-(C₁-C₆)-alkylamino bedeutet, wobei die Arylgruppen auch durch (C₁-C₆)-Alkyl oder Halogen substituiert sein können;
wobei
- R¹: nicht für Morpholino stehen kann, wenn R² Wasserstoff bedeutet.

Alkylreste und Alkenylreste können geradkettig oder verzweigt sein. Bevorzugt haben sie 1 bis 4 C-Atome. Dies gilt auch, wenn sie in Verbindung mit anderen Gruppen, z.B. als Alkoxy, Arylalkyl usw. vorliegen.

Beispiele für Alkylreste sind Methyl, Ethyl, Propyl, Isopropyl, Butyl, sek.-Butyl und tert.-Butyl.

Cycloalkyl bedeutet bevorzugt Cyclopentyl oder Cyclohexyl.

Halogen bedeutet bevorzugt Fluor, Chlor, Brom oder lod.

Aryl bedeutet bevorzugt α- oder β-Naphthyl oder Phenyl. Arylalkyl ist bevorzugt Benzyl oder Phenylethyl. Aryl-O-alkyl ist bevorzugt Phenoxymethyl oder Phenoxyethyl. Entsprechendes gilt für Aryl-S-alkyl.

Bevorzugte Reste R¹ sind Piperidino, Pyrrolidino, Dimethylamino, Diethylamino, Diisopropylamino, tert.Butyl-methylamino, tert.Butyl-ethylamino, tert.Butyl-propylamino, tert.Butylbutylamino, tert.Butyl-hydroxyethylamino, Dibenzylamino, 2-Carboxy-pyrrolidino, 2-Carboxy-piperidino, 4-Carboxypiperidino, Dicyclohexylamino, N-Methylpiperazino, 2,2-Dimethylpiperidino, 3,3-Dimethylmorpholino, 3,3-Dimethylthiomorpholino, 3,3-Dimethyl-1,1-dioxo-thiomorpholino, 3,3-Dimethyl-l-oxo-thiomorpholino, cis-2,6-Dimethylpiperidino, 2,2,6,6-Tetramethylpiperidino, 2,2-Dimethyl-N-methylsulfonyl-piperazino, 2,2-Dimethyl-N-tosylpiperazino, 2,2-Dimethyl-pyrrolidino, 2,2-Dimethyl-N-dimethylaminosulfonyl-piperazino, Ethylamino, (2-Hydroxyethyl)-amino und Cyclohexylamino. Bevorzugte Reste R² sind Wasserstoff, Methyl, Ethyl, Propyl, Isopropyl und Butyl, wobei Wasserstoff besonders bevorzugt ist.

Die Verbindungen der allgemeinen Formel I können als Hydrazone nach den für diese Substanzklasse dem Fachmann bekannten Methoden hergestellt werden (siehe z.B. Houben-Weyl, Methoden der Organischen Chemie, Band E14b/Teil I, Seite 461 ff. Thieme Verlag Stuttgart, 1990).

Beispielsweise sei die Umsetzung von Hydrazinen der allgemeinen Formel II

R¹―NH₂ (II)

mit Ketonen bzw. Aldehyden der allgemeinen Formel III 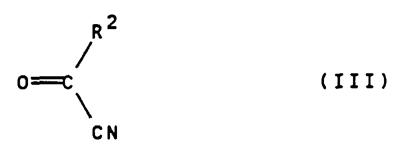 worin R¹ und R² wie oben angegeben definiert sind, genannt. Alternativ erfolgt die Herstellung der Verbindungen der allgemeinen Formel I durch Oxidation von N-Nitroso-hydrazinoacetonitrilen der allgemeinen Formel IV 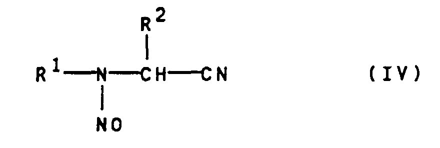 worin R¹ und R² wie oben angegeben definiert sind (siehe J. Cardiovasc. Pharmacol. 14 (Suppl. 11: S 6 - S 12 (1989)). Geeignete Oxidationshilfsmittel sind beispielsweise Sauerstoff, rotes Blutlaugensalz, CuII-Salze, Bleitetraacetat.

Die Verbindungen der allgemeinen Formel IV können in analoger Weise, wie in der EP-A 406659 oder der EP-A 40661 angegeben, durch Nitrosierung einer Verbindung der allgemeinen Formel V 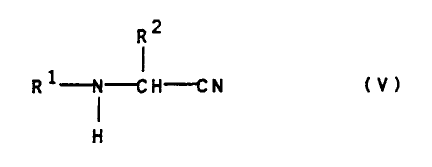 erhalten werden. Die Verbindungen der allgmeinen Formel V wiederum können in an sich bekannter Weise nach der Streckerschen Aminonitrilsynthese aus einer Verbindung der allgemeinen Formel II und einer Verbindung der allgemeinen Formel VI 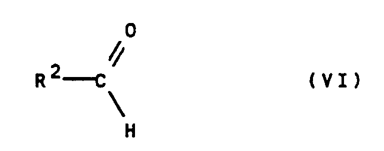 in Gegenwart von Blausäure bzw. einem geeigneten Cyanid erhalten werden.

Die Verbindungen der allgemeinen Formel I sowie N-Morpholino-iminoacetonitril besitzen wertvolle pharmakologische Eigenschaften. Durch Beeinflussung des Energiestoffwechsels der Myokardzelle, die ähnlich der des bekannten Butandionmonoxim (BDM) ist, kommt es zu einer myokardprotektiven Wirkung.

Somit können sie beim Menschen als Heilmittel eingesetzt werden, insbesondere zur Vorbeugung von Reperfusionsschäden, beispielsweise nach Lyse, bei der Angioplastie und bei Herztransplantationen. Außerdem ist ihre Verwendung in kardioplegischen Lösungen sinnvoll.

Die Dosierung kann innerhalb weiter Grenzen variieren und ist in jedem einzelnen Fall den individuellen Gegebenheiten anzupassen. Normalerweise liegt die Tagesdosis pro menschlichem Individuum bei 0,5 bis 100 mg, vorzugsweise 1 bis 20 mg.

Die Verbindungen der allgemeinen Formel I sowie N-Morpholino-iminoacetonitril können als Heilmittel für sich allein, in Mischungen untereinander und/oder in Form von pharmazeutischen Zubereitungen verabreicht werden, die eine enterale oder parenterale Anwendung gestatten und die als aktiven Bestandteil eine wirksame Dosis mindestens einer Verbindung der allgemeinen Formel I und/oder N-Morpholino-iminoacetonitril neben üblichen pharmazeutisch einwandfreien Träger- und Zusatzstoffen enthalten.

Bevorzugte pharmazeutische Zubereitungen sind parenterale Formen, also beispielsweise Injektionslösungen, weil sie es erlauben, den Wirkstoff in geeigneter Konzentration direkt an den oder zumindest in die Nähe des Wirkorts (Myokard) zu bringen.

Zubereitungen zur parenteralen Applikation enthalten 0,1 bis 10 mg, bevorzugt 0,1 bis 5 mg Verbindung der allgemeinen Formel I und/oder N-Morpholino-iminoacetonitril pro Dosiseinheit und können in separaten Dosiseinheitsformen wie z. B. Ampullen oder Vials vorliegen. Vorzugsweise werden Lösungen des Wirkstoffes verwendet, bevorzugt wäßrige Lösungen und vor allem isotonische Lösungen, aber auch Suspensionen. Diese Injektionsformen können als Fertigpräparat zur Verfügung gestellt werden oder erst direkt vor der Anwendung durch Mischen der wirksamen Verbindung, gegebenenfalls mit weiteren festen Trägerstoffen, mit dem gewünschten Lösungs-oder Suspensionsmittel zubereitet werden.

Parenterale Formen können sterilisiert sein und/oder gegebenenfalls Hilfsstoffe wie Konservierungsmittel, Stabilisatoren, Emulgatoren, Lösungsvermittler, Salze zur Regelung des osmotischen Drucks oder zur Pufferung enthalten.

Als Lösungsmittel eignen sich insbesondere Wasser, Alkohole, Glycerin, Polyole und pflanzliche Öle.

Die pharmazeutischen Zubereitungen können neben den Verbindungen der allgemeinen Formel I und/oder N-Morpholino-iminoacetonitril gegebenenfalls auch noch andere therapeutisch wirksame Stoffe enthalten.

Derartige andere therapeutisch wirksame Substanzen sind beispielsweise: β-Rezeptorenblocker, wie z.B. Propanolol, Pindolol, Metoprolol; Vasodilatatoren, wie z.B. Carbochromen; Beruhigungsmittel, wie z.B. Barbitursäurederivate, 1,4-Benzodiazepine und Meprobamat; Diuretica, wie z.B. Chlorothiazid; das Herz tonisierende Mittel, wie z.B. Digitalispräparate; blutdrucksenkende Mittel, wie z.B. Hydralazin, Dihydralazin, Ramipril, Prazosin, Clonidin, Rauwolfia-Alkaloide; Mittel, die den Fettsäurespiegel im Blut senken, wie z.B. Benzafibrat, Fenofibrat; Mittel für die Thromboseprophylaxe, wie z.B. Phenprocoumon.

Die pharmakologische Wirkung der erfindungsgemäßen Verbindungen der allgemeinen Formel I sowie des N-Morpholino-iminoacetonitrils wurden am Modell des isoliert perfundierten Rattenherzens untersucht. Die Wirkung wurde dabei mit der des 2,3-Butandionmonoxims verglichen, für die eine myokardprotektive Wirkung bereits in diesem (Schlüter et al. Am. J. Physiol. 1991; 261: H416 - H423) sowie in anderen Modellen (Siegmund et al. Am. J. Physiol. 1991; 260: H426 - H435 sowie Garcia-Dorado et al. Circulation 1992; 85: 1160 - 1174) beschrieben wurde.

Dazu wurden Herzen von 200 - 250 g schweren Wistarratten in einer temperaturkontrollierten Kammer (37°C) an ein Langendorff-Perfusionssystem gehängt. Die Kammer war während normoxischer Perfusionen mit Luft, während anoxischer mit 95 % N₂ plus 5 % CO₂ gefüllt. Die Herzen (mittleres Trockengewicht 215 mg) wurden bei 37°C mit einer Flußrate von 5 ml/min perfundiert. Für eine initiale Stabilisierungsperiode von 30 min wurden die Herzen mit einem oxygenierten salinen Medium perfundiert (Zusammensetzung in mM: 140.0 NaCl, 24.0 NaHCO₃, 2.7 KCl,
0.4 NaH₂PO₄, 1.0 MgCl₂, 1.8 CaCl₂, 5.0 Glucose, äquilibriert mit 95 % O₂ und 5 % CO₂, pH 7.4). Für anoxische Perfusionen wurde diese initiale normoxische Stabilisierungsperiode gefolgt von 60 min Perfusion, mit einem Fluß von 5 ml/min von anoxoischem Medium (Zusammensetzung wie zuvor, aber ohne Glucose und äquilibriert mit 95 % N₂ und 5 % CO₂, pH 7.4). Die anoxisch perfundierten Herzen wurden nachfolgend durch Zurückschalten auf die initialen normoxischen Perfusionsbedingungen reoxygeniert.

In Versuchen mit den Testsubstanzen wurden diese dem anoxischen Perfusat während der letzten 15 min zugefügt. Die ersten 60 min der Reoxygenationsphase wurden ebenfalls in Gegenwart der Testsubstanzen durchgeführt. Danach wurde ohne diese Substanzen die normoxische Perfusion für weitere 45 min fortgesetzt.

Die Herzschädigung wurde charakterisiert durch die Freisetzung des zytosolischen Enzyms Laktatdehydrogenase (LDH) in das Perfusat. Während der Herzperfusion wurden 1-minütige Sammelproben des Effluats gesammelt. Die Aktivität von LDH wurde auch in Homogenaten ganzer Rattenherzen gemessen (präpariert in 100 mM Phosphatpuffer, pH 7.4, mit 1 % Triton X-100). LDH wurde nach den Angaben in Bergmeyer, Bernt, Laktatdehydrogenase, in Methoden der Enzymatischen Analyse, Weinheim Verlag Chemie, 1974: 607 - 612, bestimmt. Der Enzymverlust wurde in Prozenten der initialen Gesamtgewebsaktivitäten angegeben. Die Gewebsaktivität von LDH war 601 ± Standardabweichung, n = 4 Herzen).

### Ergebnisse:

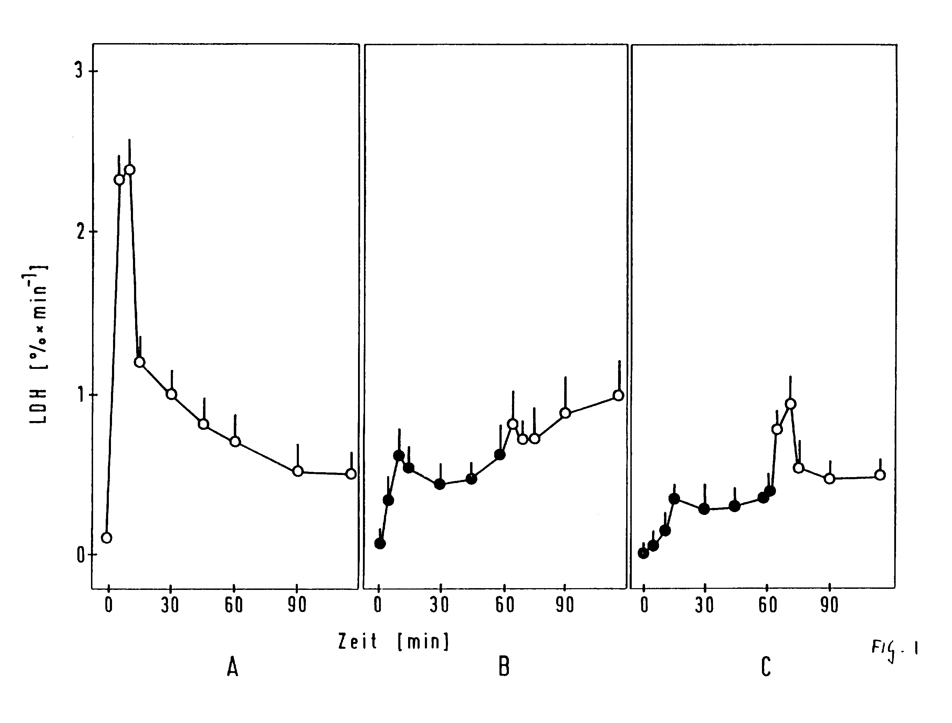

In Abbildung 1 ist der Zeitverlauf der Freisetzung von Laktatdehydrogenase aus Herzen nach 60 min Anoxie gezeigt, in Prozent der initialen Gesamtgewebsaktivität (Mittelwert ± Standardfehler; n = 4 Herzen). Die Freisetzung während der Reoxygenationsphase ist dargestellt.
A entspricht der Kontrolle, d.h. einer Reoxygenation ohne Testsubstanzen. Es zeigt sich ein großer initialer Enzymverlust.
B entspricht dem Verlauf der Reoxygenation in Gegenwart von 10 mM N-Morpholino-iminoacetonitril (geschlossene Kreise). Der große initiale Enzymverlust wird weitgehend unterdrückt. Das anschließende Auswaschen der Substanz (offene Kreise) führt zu keiner weiteren Steigerung des Enzymverlusts.
C entspricht dem Verlauf der Reoxygenation in Gegenwart von 20 mM Butandionmonoxim (BDM) (geschlossene Kreise). Auch hier ist der große initiale Enzymverlust weitgehend unterdrückt. Das anschließende Auswaschen der Substanz (offene Kreise) führt zu einer geringen initialen Steigerung des Enzymverlustes.

Die dargestellten Ergebnisse zeigen, daß die reoxygenationsbedingte Myokardschädigung durch N-Morpholino-iminoacetonitril sowie die Verbindungen der allgemeinen Formel I deutlich reduziert werden kann. Dabei ist deren Wirksamkeit etwa doppelt so hoch wie die von BDM. Berücksichtigt werden muß außerdem, daß die Toxizität von BDM zu starken Nebenwirkungen führt, die die therapeutische Anwendung stark einschränken.

### Beispiele

### 1. N-Morpholino-iminoacetonitril

Die Mischung aus 8,5 g N-Morpholino-N-nitrosoaminoacetonitril und 200 ml H₂O wird mit 16,5 g rotem Blutlaugensalz versetzt und 1 h bei RT gerührt. Das Produkt wird dann mit CH₂Cl₂ ausgeschüttelt, getrocknet (über Na₂SO₄) und nach dem Einengen im Vakuum aus tert.-Butyl-methylether umkristallisiert.
Ausbeute: 5,1 g
Fp. 73 - 5°C

Analog lassen sich herstellen:
2. N-(1,1-Dioxo-tetrahydro-1,4-thiazin-4-yl)-iminoacetonitril.
   Fp. 160 - 2°C
3. N-(1-Ethoxycarbonyl-piperazin-4-yl)-iminoacetonitril. Fp. Öl
4. N-(1-Methansulfonyl-piperazin-4-yl)-iminoacetonitril. Fp. 113 - 5°C
5. N-(tert.-Butyl-(2-hydroxyethyl)-amino)-iminoacetonitril.
   Fp. Öl
6. N-Diethylamino-iminoacetonitril.
   Fp. Öl
7. N-(2,5-cis-Dimethylpyrrolidino)-iminoacetonitril.
   Fp. Öl
8. N-(1,1-Dioxo-3,3-dimethyl-tetrahydro-1,4-thiazin-4-yl)-iminoacetonitril.
   Fp. 165 - 6°C
9. N-(1-Oxo-3,3-dimethyl-tetrahydro-1,4-thiazin-4-yl)iminoacetonitril.
   Fp. Öl
10. N-(3,3-Dimethyl-tetrahydro-1,4-thiazin-4-yl)-iminoacetonitril.
   Fp. 57 - 8°C

### 11. N-(cis-2,6-Dimethylpiperidino)-iminoacetonitril

In die Lösung von 10 g N-(cis-2,6-Dimethylpiperidino)-N-nitroso-aminoacetonitril in 150 ml Essigester wird bei 50 - 60°C Sauerstoff geleitet bis dünnschichtchromatographisch die Ausgangsverbindung nicht mehr nachgewiesen werden kann. Dann wird eingeengt und der Rückstand säulenchromatographisch (Kieselgel, LM: CH₂Cl₂/MeOH = 98 : 2) aufgearbeitet.
Ausbeute: 5,7 g
Fp. 61 - 2°C

## Patentansprüche

1. Verbindungen der allgemeinen Formel (I) 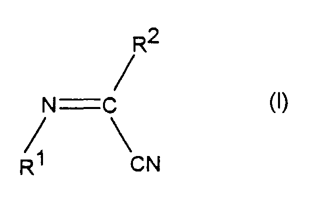 worin
R¹ eine Aminogruppe der Formel 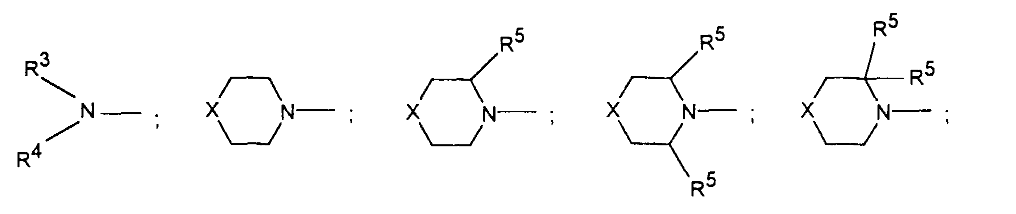 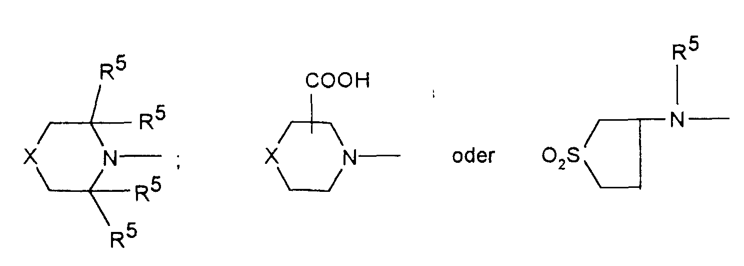 bedeutet;
R² Wasserstoff, Halogen, (C₁-C₆)-Alkyl, (C₅-C₇)-Cycloalkyl, (C₆-C₁₀)-Aryl-(C₁-C₆)-alkyl, (C₆-C₁₀)-Aryl-O-(C₁-C₆)-alkyl, (C₆-C₁₀)-Aryl-S-(C₁-C₆)-alkyl, (C₁-C₆)-Alkoxy-(C₁-C₆)-alkyl, (C₁-C₆)-Alkylthio-(C₁-C₆)-alkyl oder (C₁-C₆)-Alkenylthio-(C₁-C₆)-alkyl bedeutet;
R³ (C₁-C₆)-Alkyl, (C₁-C₆)-Alkenyl, (C₅-C₇)-Cycloalkyl, (C₆-C₁₄)-Bicycloalkyl, (C₇-C₁₆)-Tricycloalkyl, (C₁-C₆)-Alkyl-X-(C₁-C₆)-alkyl, (C₆-C₁₀)-Aryl-(C₁-C₆)-alkyl, das auch durch (C₁-C₆)-Alkyl oder Halogen substituiert sein kann, Hydroxy-(C₁-C₆)-alkyl oder einen Rest 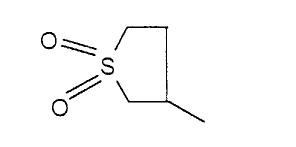
bedeutet;
R⁴ Wasserstoff bedeutet oder eine der Bedeutungen von R³ hat;
R⁵ (C₁-C₆)-Alkyl bedeutet;
X NR⁴, NSO₂R⁶, NCO₂-(C₁-C₆)-Alkyl, S(O)ₙ, O, (CH₂)ₘ oder eine einfache Bindung bedeutet;
n 0, 1 oder 2 ist;
m 1, 2 oder 3 ist; und
R⁶ (C₁-C₆)-Alkyl, (C₆-C₁₀)-Aryl, (C₁-C₆)-Alkyl-(C₆-C₁₀)-aryl, Halogen-(C₆-C₁₀)-aryl oder Di-(C₁-C₆)-alkylamino bedeutet, wobei die Arylgruppen auch durch (C₁-C₆)-Alkyl oder Halogen substituiert sein können;
wobei
R¹ nicht für Morpholino stehen kann, wenn R² Wasserstoff bedeutet.

2. Verbindungen gemäß Anspruch 1, dadurch gekennzeichnet, daß R¹ Piperidino, Pyrrolidino, Dimethylamino, Diethylamino, Diisopropylamino, tert.Butyl-methylamino, tert.-Butyl-ethylamino, tert.Butyl-propylamino, tert.Butylbutylamino, tert.Butyl-hydroxyethylamino, Dibenzylamino, 2-Carboxy-pyrrolidino, 2-Carboxy-piperidino, 4-Carboxypiperidino, Dicyclohexylamino, N-Methylpiperazino, 2,2-Dimethylpiperidino, 3,3-Dimethylmorpholino, 3,3-Dimethylthiomorpholino, 3,3-Dimethyl-1,1-dioxo-thiomorpholino, 3,3-Dimethyl-1-oxo-thiomorpholino, cis-2,6-Dimethylpiperidino, 2,2,6,6-Tetramethylpiperidino, 2,2-Dimethyl-N-methylsulfonyl-piperazino, 2,2-Dimethyl-N-tosyl-piperazino, 2,2-Dimethyl-pyrrolidino, 2,2-Dimethyl-N-dimethylaminosulfonyl-piperazino, Ethylamino, (2-Hydroxyethyl)-amino oder Cyclohexylamino bedeutet.

3. Verbindungen gemäß Anspruch 1 und/oder 2, dadurch gekennzeichnet, daß R² Wasserstoff bedeutet.

4. Verfahren zur Herstellung einer Verbindung der allgemeinen Formel I gemäß einem oder mehreren der Ansprüche 1 bis 3, dadurch gekennzeichnet, daß eine Verbindung der allgemeinen Formel IV 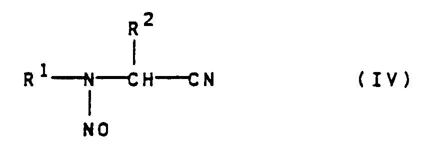 oxidiert wird.

5. Verbindung der allgemeinen Formel (I) gemäß einem oder mehreren der Ansprüche 1 bis 3 zur Anwendung als myokardprotektives Heilmittel.

6. N-Morpholino-iminoacetonitril zur Anwendung als myokardprotektives Heilmittel.

7. Pharmazeutische Zubereitung, dadurch gekennzeichnet, daß sie eine Verbindung der allgemeinen Formel I gemäß einem oder mehreren der Ansprüche 1 bis 3, pharmazeutisch einwandfreie Träger- und Zusatzstoffe sowie gegebenenfalls noch andere therapeutisch wirksame Stoffe enthält.

8. Pharmazeutische Zubereitung, dadurch gekennzeichnet, daß sie N-Morpholino-iminoacetonitril, pharmazeutisch einwandfreie Träger- und Zusatzstoffe sowie gegebenenfalls noch andere therapeutisch wirksame Stoffe enthält.

## Claims

1. A compound of the formula (I) 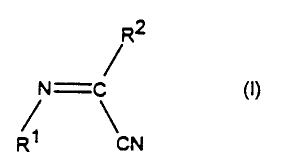 in which
R¹ is an amino group of the formula 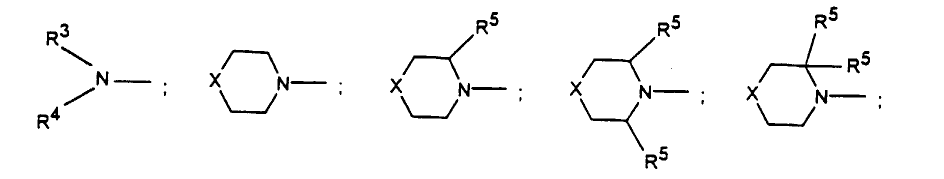 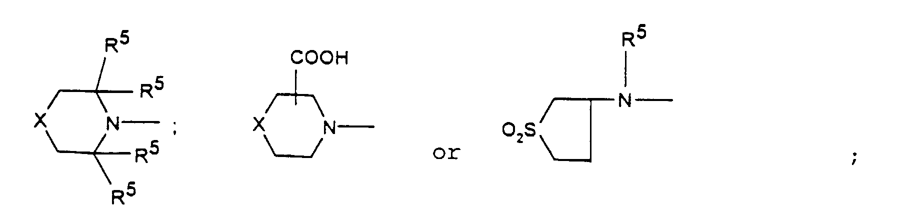
R² is hydrogen, halogen, (C₁-C₆)-alkyl, (C₅-C₇)-cycloalkyl, (C₆-C₁₀)-aryl-(C₁-C₆)-alkyl, (C₆-C₁₀)-aryl-O-(C₁-C₆)-alkyl, (C₆-C₁₀)-aryl-S-(C₁-C₆)-alkyl, (C₁-C₆)-alkoxy-(C₁-C₆)-alkyl, (C₁-C₆)-alkylthio-(C₁-C₆)-alkyl or (C₁-C₆)-alkenylthio-(C₁-C₆)-alkyl ;
R³ is (C₁-C₆)-alkyl, (C₁-C₆)-alkenyl, (C₅-C₇)-cycloalkyl, (C₇-C₁₄)-bicycloalkyl, (C₇-C₁₆)-tricycloalkyl, (C₁-C₆)-alkyl-X-(C₁-C₆)-alkyl, (C₆-C₁₀)-aryl-(C₁-C₆)-alkyl which can also be substituted by (C₁-C₆)-alkyl or halogen, is hydroxy-(C₁-C₆)-alkyl or is a radical 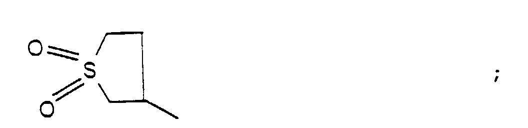
R⁴ is hydrogen or has one of the meanings of R³;
R⁵ is (C₁-C₆)-alkyl;
X is NR⁴, NSO₂R⁶, NCO₂-(C₁-C₆)-alkyl, S(O)ₙ, O, (CH₂)ₘ or a single bond;
n is 0, 1 or 2;
m is 1, 2 or 3; and
R⁶ is (C₁-C₆)-alkyl, (C₆-C₁₀)-aryl, (C₁-C₆)-alkyl-(C₆-C₁₀)-aryl, halo-(C₆-C₁₀)-aryl or di-(C₁-C₆)-alkylamino, where the aryl groups can also be substituted by (C₁-C₆)-alkyl or halogen;
where
R¹ is not morpholino if R² is hydrogen.

2. A compound as claimed in claim 1, wherein R¹ is piperidino, pyrrolidino, dimethylamino, diethylamino, diisopropylamino, tert-butylmethylamino, tert-butylethylamino, tert-butylpropylamino, tert-butylbutylamino, tert-butylhydroxyethylamino, dibenzylamino, 2-carboxypyrrolidino, 2-carboxypiperidino, 4-carboxypiperidino, dicyclohexylamino, N-methylpiperazino, 2,2-dimethylpiperidino, 3 ,3-dimethylmorpholino, 3,3-dimethylthiomorpholino, 3,3-dimethyl-1,1-dioxothiomorpholino, 3,3-dimethyl-1-oxothiomorpholino, cis-2,6-dimethylpiperidino, 2,2,6,6-tetramethylpiperidino, 2,2-dimethyl-N-methylsulfonylpiperazino, 2,2-dimethyl-N-tosylpiperazino, 2,2-dimethylpyrrolidino, 2,2-dimethyl-N-dimethylaminosulfonylpiperazino, ethylamino, (2-hydroxyethyl)amino or cyclohexylamino.

3. A compound as claimed in claim 1 and/or 2, wherein R² is hydrogen.

4. A process for the preparation of a compound of the formula I as claimed in one or more of claims 1 to 3, which comprises oxidizing a compound of the formula IV

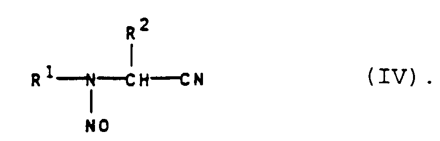

5. A compound of the formula (I) as claimed in one or more of claims 1 to 3 for use as a myocardial-protective therapeutic.

6. N-Morpholinoiminoacetonitrile for use as a myocardial-protective therapeutic.

7. A pharmaceutical preparation which comprises a compound of the formula I as claimed in one or more of claims 1 to 3, pharmaceutically innocuous excipients and additives and also, if desired, other therapeutically active substances.

8. A pharmaceutical preparation which comprises N-morpholinoiminoacetonitrile, pharmaceutically innocuous excipients and additives and also, if desired, other therapeutically active substances.

## Revendications

1. Composés de formule générale (I) 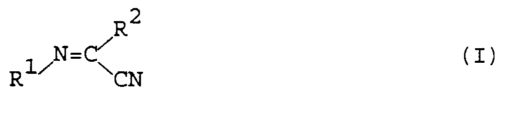 dans laquelle
R¹ représente un groupe amino de formule 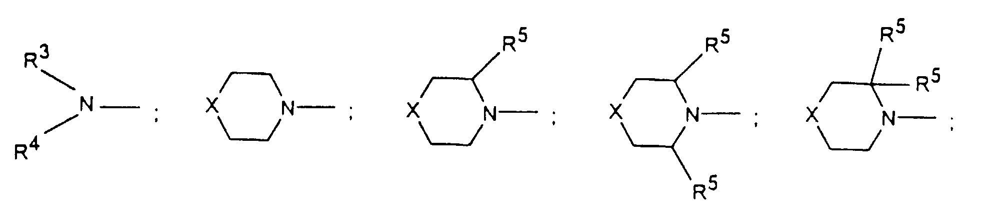 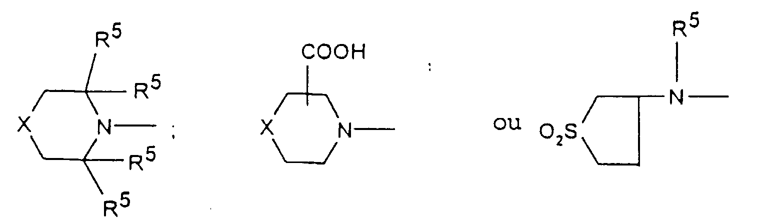
R² représente un atome d'hydrogène ou d'halogène ou un groupe alkyle en C₁-C₆, cycloalkyle en C₅-C₇, aryl-(C₆-C₁₀)-alkyle(C₁-C₆), aryl(C₆-C₁₀)-O-alkyle (C₁-C₆), aryl(C₆-C₁₀)-S-alkyle(C₁-C₆), alcoxy(C₁-C₆)-alkyle-(C₁-C₆), alkyl(C₁-C₆)thio-alkyle(C₁-C₆) ou alcényl-(C₁-C₆)thio-alkyle(C₁-C₆) ;
R³ représente un groupe alkyle en C₁-C₆, alcényle en C₁-C₆, cycloalkyle en C₅-C₇, bicycloalkyle en C₇-C₁₄, tricycloalkyle en C₇-C₁₆, alkyl(C₁-C₆)-X-alkyle(C₁-C₆), aryl(C₆-C₁₀)-alkyle(C₁-C₆), qui peut également être substitué par un atome d'halogène ou par un groupe alkyle en C₁-C₆, ou représente un groupe hydroxyalkyle en C₁-C₆ ou un radical 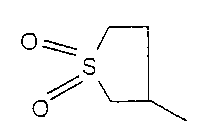
R⁴ représente un atome d'hydrogène ou a l'une des significations de R³;
R⁵ représente un groupe alkyle en C₁-C₆;
X représente un groupe NR⁴, NSO₂R⁶, NCO₂-alkyle(C₁-C₆), S(O)ₙ, O, (CH₂)ₘ ou une liaison simple;
n est 0, 1 ou 2;
m est 1, 2 ou 3; et
R⁶ représente un groupe alkyle en C₁-C₆, aryle en C₆-C₁₀, alkyl(C₁-C₆)-aryle(C₆-C₁₀), halogénoaryle en C₆-C₁₀ ou dialkyl(C₁-C₆)amino, les groupes aryle pouvant également être substitués par des atomes d'halogène ou des groupes alkyle en C₁-C₆;
R¹ ne pouvant pas représenter le groupe morpholino lorsque
R² représente un atome d'hydrogène.

2. Composés selon la revendication 1, caractérisés en ce que R¹ représente le groupe pipéridino, pyrrolidino, diméthylamino, diéthylamino, diisopropylamino, tert-butylméthylamino, tert-butyléthylamino, tert-butylpropylamino, tert-butylbutylamino, tert-butylhydroxyéthylamino, dibenzylamino, 2-carboxypyrrolidino, 2-carboxypipéridino, 4-carboxypipéridino, dicyclohexylamino, N-méthylpipérazino, 2,2-diméthylpipéridino, 3,3-diméthylmorpholino, 3,3-diméthylthiomorpholino, 3,3-diméthyl-1,1-dioxothiomorpholino, 3,3-diméthyl-1-oxothiomorpholino, cis-2,6-diméthylpipéridino, 2,2,6,6-tétraméthylpipéridino, 2,2-diméthyl-N-méthylsulfonylpipérazino, 2,2-diméthyl-N-tosylpipérazino, 2,2-diméthylpyrrolidino, 2,2-diméthyl-N-diméthylaminosulfonylpipérazino, éthylamino, (2-hydroxyéthyl)amino ou cyclohexylamino.

3. Composés selon la revendication 1 et/ou la revendication 2, caractérisés en ce que R² représente un atome d'hydrogène.

4. Procédé pour la préparation d'un composé de formule générale I selon une ou plusieurs des revendications 1 à 3, caractérisé en ce que l'on oxyde un composé de formule générale IV

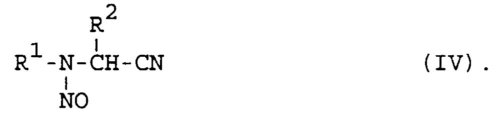

5. Composé de formule générale (I) selon une ou plusieurs des revendications 1 à 3, pour utilisation en tant que médicament protecteur du myocarde.

6. N-morpholino-iminoacétonitrile pour utilisation en tant que médicament protecteur du myocarde.

7. Composition pharmaceutique, caractérisée en ce qu'elle contient un composé de formule générale I selon une ou plusieurs des revendications 1 à 3, des véhicules et additifs pharmaceutiquement acceptables ainsi qu'éventuellement encore d'autres substances thérapeutiquement actives.

8. Composition pharmaceutique, caractérisée en ce qu'elle contient du N-morpholino-iminoacétonitrile, des véhicules et additifs pharmaceutiquement acceptables ainsi qu'éventuellement encore d'autres substances thérapeutiquement actives.
